# EUROPEAN PATENT APPLICATION

(11) **EP 4 289 998 A2**
(43) Date of publication of application: **13.12.2023**
(21) Application number: 23206797.5
(22) Date of filing: 15.02.2018
(51) Int. Cl.: D01F 4/02

(54) **A FILAMENT AND YARN PRODUCED ON THE BASIS OF A NATURAL PROTEIN**

(30) Priority: 15.02.2017 FI 20175134
(62) Divisional of application: 18705904.3
(71) Applicant: Ecco Sko A/S, 6261 Bredebro (DK); Spinnova Oy, 40800 Vaajakoski (FI)
(72) Inventor: GØGSIG, Thomas, 6261 Bredebro (DK); LIUKKONEN, Johanna, 40530 Jyväskylä (FI); HAAVISTO, Sanna, 40100 Jyväskylä (FI); SELENIUS, Pasi, 41400 Lievestuore (FI); SALMELA, Juha, 41340 Laukaa (FI); PORANEN, Janne, 40950 Muurame (FI); SALMINEN, Arto, 40420 Jyskä (FI); MYLLYS, Markko, 40700 Jyväskylä (FI); VENTO, Pia, 40800 Vaajakoski (FI); BJÖRKLUND, Karri, 40700 Jyväskylä (FI); PETRUSIC, Stojanka, 6261 Bredebro (DK)
(74) Representative: Patentgruppen A/S

(57) **Abstract**

The invention relates to a reconstructed filament wherein the reconstructed filament comprises natural protein fibrils and an additive.

## Description

### Field of the invention

The invention relates to a filament and yarn produced on the basis of a natural protein

### Background of the invention

There is an increasing interest to replace or reduce the use of fossil-based materials with bio-based and renewable materials so as to support environmental sustainability. The development and use of renewable raw materials can help to reduce the usage of fossil resources. A challenge is however that the natural fiber sources, when used exhibits inferior properties e.g., in relation to strength and other relevant properties when used e.g., in relation to yarn spun from these fibers.

### Summary of the invention

The invention relates to a reconstructed filament wherein the reconstructed filament comprises natural protein fibrils and an additive.

In the present context, the additive designates an additive which is bonding distinct natural protein-based fibrils. Additive(s) added onto the surface of the reconstructed protein fiber filament is(are) for the purpose of calculation of the claimed weight percent not included in the calculation as such additives are functionally not regarded as a part of the fiber structure. Additives within the present context thus refer to compound(s) forming part of the fiber structure. In other words, additives functioning as a bonding agent between distinct natural protein-based fibrils are functionally different from additives added onto the surface of the reconstructed protein fiber filament. Additives not forming a part of the fiber structure and not forming part of or facilitating the bonding structure between the natural protein fiber fibrils may e.g., include different types of lubricants added to the outer surface of the reconstructed staple fiber.

Additives forming a part of the calculation facilitates the bonding structure between the natural protein fiber fibrils of the. These will be described in detail below.

The term "reconstructed filament" in the present context defines that the filament as such has been made by combining protein fibrils by means of additives and the reconstructed filament will thus differ in structure from a corresponding natural fiber if such natural counterpart exists. In the example of a reconstructed filament based on collagen fibrils obtained from natural collagen fibers, a reconstructed collagen filament may thus have properties not so different from the originating natural collagen fiber.

The applied protein fibrils will primarily or solely be fibrils obtained by mechanically dividing the source of protein fibers and the additives applied may additives promoting the bonding between the fibrils into the filament.

The structure of the filament according to an embodiment of the invention cannot be converted or disassembled into monolithic sub-structures, such as fiber bundles of natural fibrous polymers, strips or ribbons, using mechanical cutting, untwisting, grinding or chemical separating means. Disintegration of the filament yields only individual fibrous elements. In an example, the filament may be disintegrated through breaking the hydrogen bonds, for example, by using water or another aqueous solution so as to return the monofilament type fiber structure into separate fibrous elements such as primary protein fibrils.

An "originating natural protein fiber" would in the present context refer to the source by means which the protein fibrils are produced by means of mechanical dividing of the natural fiber source, the "originating natural protein fiber".

In an embodiment of the invention, the reconstructed filament comprises natural protein fibrils and an additive, wherein the reconstructed filament comprises natural protein fibrils in an amount of between 70 and 99.9 % by weight of the filament and an additive between 0.1 and 30 % by weight of the filament.

It should again be noted that the given weight percentage is given with reference to a reconstructed filament excluding optional surface treatment compound. Fractions of the additives forming part of the reconstructed filament or staple fiber and used during the reconstructing of the protein-based filament may of course appear at the surface and these fractions will be included in the complete calculation. Typically, it is thus understood that compounds used for post-treatment of a reconstructed filament or staple fiber will thus not be regarded as a part of the filament or staple fiber. In other words, a filament for example constituted and reconstructed to include 90% by weight of natural protein fibrils and 10% by weight of additives will refer to a reconstructed filament excluding any optional post treatment.

In an embodiment of the invention, the reconstructed filament comprises elastin fibrils.

In an embodiment of the invention, the reconstructed filament comprises resilin fibrils.

In an embodiment of the invention, the reconstructed filament comprises keratin fibrils.

In an embodiment of the invention, said protein fibrils comprise collagen fibrils obtained from natural collagen fibers.

In an embodiment of the invention, the natural protein fibrils of the reconstructed filament consist of natural protein fibrils.

In an embodiment of the invention, the natural protein fibrils of the reconstructed filament consist of natural collagen fibrils.

In an embodiment of the invention, the natural protein fibrils originate from mechanically subdivided natural protein fibers.

In an embodiment of the invention, the length of the originating natural protein fibers are different from the length of the reconstructed filament.

Wherein the sizes of the originating natural protein fibers are different from the length of the reconstructed filament.

In an embodiment of the invention, the reconstructed filament comprises additive(s). The additive(s) comprise(s) at least one of the following: alginate, alginic acid, pectin, carrageenan, carboxymethyl cellulose, starch, polyacrylamide, polyethylene oxide, laccase, transglutaminase, polyphenol, glutaraldehyde, gelatine, casein, glucose, Tris-HCl, phosphate and vinyl acetate.

The additives may according to an embodiment of the invention consist of one or more of the following additives or any combination thereof: alginate, alginic acid, pectin, carrageenan, carboxymethyl cellulose, starch, polyacrylamide, polyethylene oxide, laccase, transglutaminase, polyphenol, glutaraldehyde, gelatine, casein, glucose, Tris-HCl, phosphate and vinyl acetate.

In an embodiment of the invention, the reconstructed filament comprises additive consisting of carboxymethyl cellulose (CMC) and/or starch.

In an embodiment of the invention, the reconstructed filament comprises additive consisting of carboxymethyl cellulose (CMC) and polyethylene oxide.

In an embodiment of the invention, the reconstructed filament comprises at most 85% by weight of the natural collagen fibrils, at most 10% by weight the additive carboxymethyl cellulose and at most 5% by weight of the additive polyethylene oxide.

In an embodiment of the invention, the reconstructed filament comprises at most 85% by weight of the natural collagen fibrils, at most 15% by weight of the additive alginate.

In an embodiment of the invention, the breaking strength of the reconstructed filament is at least 6 cN/tex, wherein cN/tex stands for linear mass density-related tenacity.

The breaking strength of 6 cN/tex primarily relates to filament comprising mainly of collagen fibrils or other protein fibrils, all of natural origin, i.e., obtained from natural proteins.

Some significant breaking strengths of fibers are 35-60 cN/tex for polyester fiber, 15-40 cN/tex for cotton and 12-18 cN/tex for wool.

In an embodiment of the invention the reconstructed filament wherein the fibrils consist of proteins and wherein the breaking strength of the reconstructed filament is at least 6 cN/tex, wherein cN/tex stands for linear mass density-related tenacity.

According to an embodiment of the invention, the breaking strength of the reconstructed filament is at least 8 cN/tex, wherein cN/tex stands for linear mass density-related tenacity.

In an embodiment of the invention, the breaking strength of the reconstructed filament is at least 10 cN/tex, wherein cN/tex stands for linear mass density-related tenacity.

According to a very advantageous embodiment of the invention, the breaking strength of the reconstructed filament is at least 12 cN/tex, wherein cN/tex stands for linear mass density-related tenacity.

This breaking strength is very attractive as this breaking strength compares with the available strength of e.g., natural wool fibers, but now also even obtainable on the basis of a completely different protein fiber, namely e.g., the collagen fiber.

In an embodiment of the invention, the elongation of the reconstructed filament is at least 5%, wherein elongation is specified as a percentage of the starting length.

According to an embodiment of the invention, the elongation of the reconstructed filament is at least 10%, wherein elongation is specified as a percentage of the starting length.

According to an embodiment of the invention, the elongation of the reconstructed filament is at least 15%, wherein elongation is specified as a percentage of the starting length.

According to a specific and very advantageous embodiment of the invention, the above elongation properties may be obtained through use of natural collagen fibers as the main or sole source of protein fibrils for the reconstructed filament.

The elastic elongation is of decisive importance since e.g., textile products without elasticity would hardly be usable. They must be able to deform (e.g., at knee or elbow) in order to withstand high loading (and also during processing), but they must also return to shape. The fiber elongation should therefore be at least 1-2% (glass fibers), and preferably slightly more. The greater crease-resistance of wool compared with cotton arises, for example, from the difference in their elongation where cotton is 6-10% and wool 25-45%.

In an embodiment of the invention, the natural protein fibrils comprises fibrils of tanned hides.

Tanning is the conversion of a putrescible organic material into a stable material that resists putrefaction by spoilage bacteria.

In the specific context, tanning may refer to a method whereby a hide from an animal is treated chemically to convert hide to leather. Tanning methods may thus involve use of different types of relevant chemicals known within the art. Thus, the protein fibrils used for the reconstructing of a filament, or of course a staple fiber within the scope of the invention may include protein fibrils which has been pretreated with chemical for another purpose than the intended reconstruction. It has thus surprisingly been shown that collagen fibrils scraped or loosened from tanned skin may be gathered into a reconstructed filament despite this pretreatment.

In an embodiment of the invention, the natural protein fibrils comprises fibrils of wet-blue.

In an embodiment of the invention, the natural protein fibrils comprises fibrils of non-tanned hides.

The invention further relates to a reconstructed staple fiber manufactured on the basis of a reconstructed filament according to the invention.

In an embodiment of the invention, the lengths of the originating natural protein fibers are different from the length of the reconstructed staple fiber.

The invention further relates to a wool comprising of a plurality of staple fibers made of the reconstructed filament of the invention, wherein the natural protein fibrils originate from mechanically subdivided natural protein fibers.

In an embodiment of the invention, the staple fibers are produced on the basis of said reconstructed filaments.

The division into staple fibers of discrete lengths may e.g., be performed during or subsequent to the reconstructing of the inventive filament.

The staple fibers made from the inventive filament and comprised in the wool may be of substantially the same length. E.g., with a variation in length of +/-5%.

The staple fibers may also be of different lengths and thus should, when basing a subsequent yarn spinning on the basis of the wool, be reflected in the length distribution of staple fibers in the wool. It is desired that a substantial part of the staple fibers should be at least 3mm, such as at least 5mm, such as at least 10mm, such as at least 15 mm, such as at least 20mm.

The invention furthermore relates to a yarn spun from staple fibers of reconstructed filament according to the invention.

The invention furthermore relates to a yarn comprising a plurality of spun staple fibers based on natural collagen, wherein the yarn comprises collagen staple fibers having a length of at least 10mm.

In an embodiment, the yarn comprises collagen staple fibers having a length of 6-80 mm.

According to an advantageous embodiment of the invention, it is possible to obtain a usable yarn wherein the yarn is spun from an amount of longer staple fibers based on collagen, here at least 15 mm in length. Obviously, the designation of the length of such long natural collagen based staple fibers does not preclude the use of shorter staple fibers in the same yarn. It is just important that a sufficient amount of the natural collagen-based fibers are longer than the designated length of 15 mm.

According to an advantageous embodiment of the invention, it is possible to obtain a usable yarn wherein the yarn is spun from an amount of longer staple fibers based on collagen, here at least 20mm in length. Obviously, the designation of the length of such long natural collagen-based fibers does not preclude the use of shorter staple fibers in the same yarn. It is just important that a sufficient amount of the natural collagen-based fibers are longer than the designated length of 20mm.

In an embodiment of the invention, the staple fibers comprise length of 6-80 mm, preferably 30-70 mm.

According to an embodiment of the invention, the yarn is spun from a plurality of staple fibers based on natural collagen, wherein the yarn comprises collagen staple fibers having an average length of at least 5mm.

According to an embodiment of the invention, the yarn is spun from a plurality of staple fibers based on natural collagen, wherein the yarn comprises collagen staple fibers having an average length of at least 10mm.

According to an embodiment of the invention, the yarn is spun from a plurality of staple fibers based on natural collagen, wherein the yarn comprises collagen staple fibers having an average length of at least 15mm.

In an embodiment of the invention, the breaking strength of the staple fibers of the yarn is at least 7 cN/tex, wherein cN/tex stands for linear mass density-related tenacity.

In an embodiment of the invention, the breaking strength of the staple fibers of the yarn is at least 9 cN/tex, wherein cN/tex stands for linear mass density-related tenacity.

In an embodiment of the invention, the breaking strength of the staple fibers of the yarn is at least 12 cN/tex, wherein cN/tex stands for linear mass density-related tenacity.

In an embodiment of the invention, the breaking strength of the staple fibers of the yarn is at least 15 cN/tex, wherein cN/tex stands for linear mass density-related tenacity.

In an embodiment of the invention, the breaking strength of the staple fibers of the yarn is at least 18 cN/tex, wherein cN/tex stands for linear mass density-related tenacity.

According to an embodiment of the invention, the natural collagen fibril yarn has a wherein the breaking strength of the staple fibers of the yarn is at least 8 cN/tex, wherein cN/tex stands for linear mass density-related tenacity.

In an embodiment of the invention, the yarn exhibits linear mass density of more than 100 dtex and elongation of at least 10%.

In an embodiment of the invention, the yarn exhibits linear mass density from about 100 to 500 dtex and elongation of at least 10%.

The invention further relates to the use of the filament, the staple fiber and/or the yarn of the invention in textile and/or shoe industry.

The invention additionally relates to an item comprising the filament, the staple fiber and/or the yarn of the invention, the item comprising fabric, woven material, nonwoven material, clothing or footwear product.

### The figures

The invention will be described in the following with reference to the drawings where,
**Fig. 1** shows an illustration of a fiber product comprising fibrous elements.
**Fig. 2** shows an example of mechanical properties of fiber products.
**Fig. 3** shows an example of staple fibers.
**Fig. 4** shows an example of an aqueous suspension comprising fibrous elements of natural protein.
**Fig. 5** shows an example of mechanical properties of fiber products.
**Fig. 6** illustrates a method for manufacturing a fiber based raw wool according to an embodiment of the invention.
**Fig. 7** illustrates a method for manufacturing a fiber based raw wool according to an embodiment of the invention.
**Fig. 8** illustrates an apparatus for manufacturing a fiber based raw wool according to an embodiment of the invention.
**Fig. 9** illustrates a cylinder according to an embodiment.
**Fig. 10** illustrates an apparatus for manufacturing a fiber based raw wool according to an embodiment of the invention and where,
**Fig. 11** illustrates a belt according to an embodiment.

### Detailed description

In this description and claims, the percentage values relating to an amount of material are percentages by weight (wt.%) based on the total weight of the filament or the staple fiber in question unless otherwise indicated. Word "comprising" may be used as an open term, but it also includes the closed term "consisting of'.

In the current application term "fiber product", when used refers to fibrous filaments or staple fibers.

The term "staple fiber" refers to fibers of discrete length, such as shortened fibrous filaments. The staple fibers include individual fibrils interlocked together. The fibrous filament may be shortened into staple fibers of a certain length.

The staple fibers may be further processed in order to provide an item, such as a yarn or a non-woven material.

The fibrous filaments and/or staple fibers may be used for forming items of textile and/or shoe industry. Items include, for example, yarns, fabrics, woven materials, non-woven materials, clothing and footwear products.

Fibrils, the building block of fibers, may comprise natural fibrous proteins. Natural fibrous protein may originate from natural resources, such an animal based raw material source.

### PROTEINS

Natural fibrous proteins include structural proteins, such as collagen, elastin, keratin, silk and resilin. Natural proteins, also referred to as native proteins, have not undergone any chemical modification. Fibrous proteins may also be referred to as scleroproteins. Structural proteins exhibit long-range ordered molecular secondary structures that arise due to the highly repetitive primary amino acid sequences within the proteins. Secondary structures include twisted helices (keratins), beta-pleated sheets (spider silk), coiled coils (bee silk), beta-spirals (elastin), beta-turns (resilin) and triple helices (collagen). Proteins may be physically or chemically treated, such as gelatin.

Collagen may have macromolecular structure repeating amino acid sequences (GPX)n and glycine(G)-X-hydroxyproline(HYP). (GPX)n is the most common amino acid sequence in collagen. In the sequence X refers to any amino acid other than glycine, proline or hydroxyproline. The amino acid composition of collagen is atypical for proteins, particularly with respect to its high hydroxyproline content. Collagens are the most abundant proteins found in tissues of animals. Collagen is main structural protein in extracellular space in the connective tissues including tendons, ligaments and skin. There are several types of collagen. Type I (ski, tendon, bone), II (hyaline cartilage), III (reticulate), V (cell surfaces, hair, placenta) and XI (cartilage) being fibrillar. A single collagen molecule, tropocollagen, is used to make up larger collagen aggregates, such as fibrils. A collagen molecule is approximately 300 nm long and 1.5 nm in diameter. It is made up of three polypeptide strands (called alpha peptides), each of which has the conformation of a left-handed helix. These three left-handed helices are twisted together into a right-handed triple helix or "super helix", a cooperative quaternary structure stabilized by many hydrogen bonds. With fibrillary collagen each triple-helix associates into a right-handed super-super-coil referred to as a collagen microfibril. Each microfibril is interlocked with its neighbouring microfibrils so as to form collagen fiber. Collagen fibers are further forming collagen fiber bundles. Collagen fibrils diameter should range from 10 to 500 nm, length around 10 µm.

If collagen is partly irreversibly hydrolyzed (e.g., by heat and chemicals), it is termed gelatin. Gelatin is modified protein.

Keratin filaments are present, for example, in epithelial cells. Keratin is insoluble in water and organic solvents. Keratin is the main structural material e.g., in the outer layer of human skin. It is also main component of the wool fiber. Keratin is basically formed by polypeptide chains, which coil into a- helices with sulfur cross-links or bond into β-sheets linked by hydrogen bonding.

Elastin may have macromolecular structure repeating amino acid sequences [GVGVP]n. Elastin is a highly elastic protein in connective tissue. Different elastin proteins can be isolated from animal tissues such as skin. Elastin is composed of soluble precursor tropoelastin protein molecules. Tropoelastin contains primarily, glycine (G) and valine (V). It further contains modified alanine (A) and proline (P) residues. Tropoelastin is a 50-70kDa protein molecule that is highly cross-linked to form an insoluble complex. To make mature elastin fibers, the tropoelastin molecules are cross-linked via their lysine residues with desmosine and isodesmosine cross-linking molecules. The enzyme performing the crosslinking is lysyl oxidase resulting in formation of desmosine cross-links. The elastin consists of alternating hydrophobic and hydrophilic domains, which are encoded by separate exons, so that the domain structure of tropoelastin reflects the exon organization of the gene. The hydrophobic domains of elastin are rich in non-polar amino acids, with common repeat sequences such as [GVGVP]n, while hydrophilic domains contain a high content of lysine that are involved in elastin cross-linking and help to stabilize the structure.

Spider silk and silkworm silk may have macromolecular structure repeating amino acid sequences [GAGAGS]n, or [AAAAA]n. Silk proteins are fibrous proteins reconstructed and spun into fibers by silkworms and spiders. Fibroin is an insoluble natural protein present in silk. In addition, silk includes sericin surrounding the fibroin.

Resilin may have macromolecular structure repeating amino acid sequences [GGRPSDSYGAPGGGN]n. Resilin is an elastomeric protein found in many insects.

The above-mentioned proteins may all constitute the source of fibrils when reconstructing filament or stable fibers.

### FIBER FILAMENT

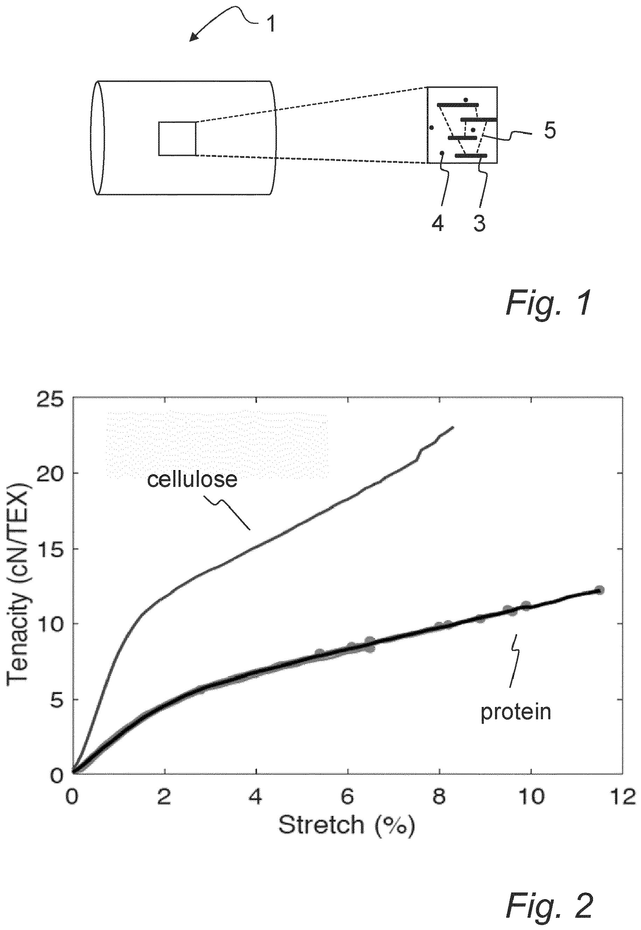

Fig. 1 shows a staple fiber 1 according to an embodiment of the invention.

The illustrated staple fiber 1 include fibrous protein fibrils 3in contact with an additive 4. The fibrils 3 are connected to each other, for example via hydrogen bonds 5 and/or mechanical interlocking 5 so as to form a coherent fibrous structure, the staple fiber 1.

### RECONSTRUCTED FILAMENT OR STAPLE FIBER

The filament or staple fiber may comprise fibrous elements of fibrils of natural fibrous collagen.

When using these fibrils for the purpose of reconstructing a filament or a staple fiber on the basis of these, the fibrils may be obtained by mechanical division of the natural fiber source.

In an example a reconstructed filament or a staple fiber comprises fibrous elements of natural fibrous proteins, such as fibrils of collagen, elastin and/or resilin.

According to an example, a reconstructed filament or a staple fiber product comprises fibrous elements of fibrous protein(s), i.e., fibrils. Preferably the fibrous proteins are natural structural proteins. In an example, the fiber product includes fibrils of at least one of the following fibrous proteins: collagen, elastin, and resilin. Preferably the fiber product includes at least fibrous elements of collagen fibrils.

In an example, a reconstructed filament or staple fiber includes at least 50%, for example between 50 and 100%, or between 70 and 100% fibrils which are fibrils of fibrous protein(s). An amount of the fibrils of fibrous protein(s), such as collagen fibrils, being at least 50 wt.% may have effect on elasticity of the fiber product.

In an example, a reconstructed fiber product comprises fibrils between 70 and 99.9 wt.% of a first fibril type of at least one of the following natural fibrous proteins: collagen, elastin, and resilin.

In addition to the fibrils, the reconstructed filament or staple fiber includes additives. The total amount of additive(s) may be between 0.01 and 30 wt.%, preferably from 0.05 to 20 wt.% or from 0.1 to 15 wt.%.

Furthermore, the length of the reconstructed staple fibers may in principle made to fit requirements e.g., to a wool made up of the produced staple fibers, the strength or stretch of the finally produced yarn, etc.

### ADDITIVES

A reconstructed filament of fiber includes additive(s) like rheology modifier(s), binder(s), cation active reagent(s), crosslinking agent(s), dispersion agent(s), pigment(s), and/or other modifier(s). The applied additives may be designed to fit into the specific application, e.g., considering the natural protein.

The fibrous filaments and staple fibers may comprise additive(s), such as alginate, alginic acid, pectin, carrageenan, anionic polyacrylamide (APAM), polyethylene oxide (PEO), carboxymethyl cellulose (CMC), starch, enzymes (such as laccase, transglutaminase), polyphenols, glutaraldehyde, gelatine, casein, glucose, Tris-HCl, phosphate or a combination of such.

In an example, the fiber product such as a filament or a staple fiber includes between 0.1 and 30 wt.% or preferably between 0.1 to 15 wt.% additives, which is at least one of the following: alginate, alginic acid, pectin, carrageenan, carboxymethyl cellulose (CMC), starch, anionic polyacrylamide (APAM), cationic polyacrylamide (CPAM), polyethylene oxide (PEO) enzymes (such as laccase, transglutaminase), polyphenols, glutaraldehyde, gelatine, casein, glucose, Tris-HCl, phosphate and resins like vinyl acetate.

According to an example, a reconstructed fiber product such as a filament or a staple fiber includes an additive of carboxymethyl cellulose (CMC) between 0.1 and 15 wt.%.

According to an example, a reconstructed fiber product such as a filament or a staple fiber includes 85 wt.% of fibrils of collagen and total amount 15% of additives. The additives are as follows: 10 wt.% of carboxymethyl cellulose (CMC) and 5 wt.% of polyethylene oxide (PEO).

During manufacturing of the reconstructed fiber product, such as a filament or a staple fiber, the additive, such as an alginate, may have effect on forming hydrogel. In addition, alginate may act as a binder in the fiber product structure. Alginate may cause crosslinking, which may have effect on binding of fibrils of the fiber product. Alginate matrix may crosslink around the fibrils and enclose the fibrils. In an example, the fiber product, such as fibrous filaments and staple fibers, may comprise between 0.1 and 15 wt.% of binder, such as alginate.

The reconstructed fibrous filaments and staple fibers may comprise a crosslinking agent and reagent pair. A crosslinking agent may be arranged to react with the reagent at the nozzle exit. Crosslinking reaction between the crosslinking agent and reagent pair creates an aqueous hydrogel and thereby influences the initial strength of the fibrous suspension. A fiber product may comprise 0-25 wt.%, preferably between 0.5 and 25% or between 1 and 10 wt.% of the crosslinking agent. The crosslinking may have effect on improving the properties of the reconstructed fiber, such as wet and/or dry strength. It may also have effect on stretch and washability of the fiber product.

The fibrous filaments and staple fibers may comprise a dispersion agent. A dispersion agent may comprise anionic long chained polymer, carboxymethyl cellulose (CMC), starch, anionic polyacrylamides (APAM), cationic polyacrylamide (CPAM),or a combination of such. A fibrous filament may comprise 0-20 wt.%, preferably between 0.5 and 25% or between 1 and 10 wt.% of a dispersion agent. The dispersion agent may have effect on shear strength of the fiber product. In an example, the fiber product may comprise between 0.1 and 10 wt.% of additive, such as CMC (carboxymethyl cellulose) and/or starch.

### MANUFACTURING OF A RECONSTRUCTED FILAMENT OR STAPLE FIBER

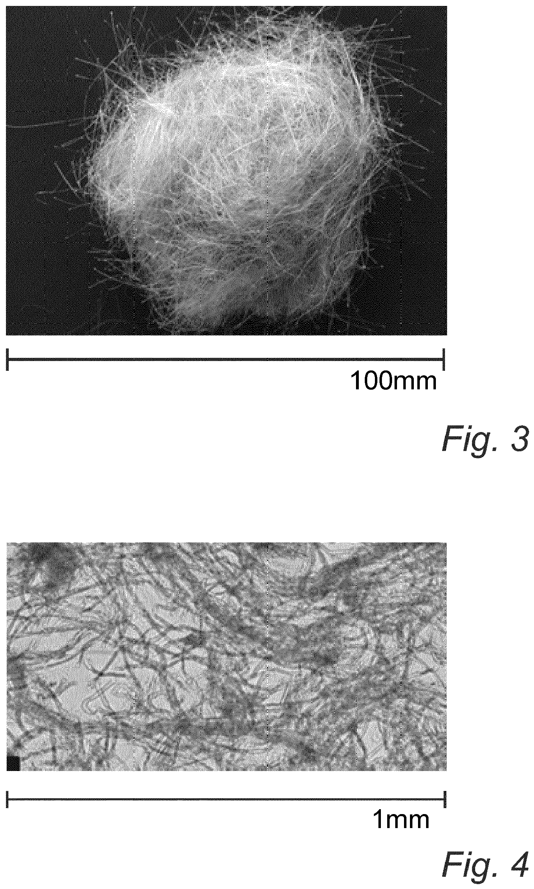

A fibrous suspension is provided. The term "suspension" refers here to a heterogeneous fluid containing solid particles, such as fibrils. It encompasses also slurries and dispersions. Typically, solid particles are in aqueous liquid. The fibrous suspension comprises an aqueous suspension of natural fibrous polymers, such as fibrils of fibrous proteins. An example of a suspension comprising fibrils of natural fibrous proteins is shown in fig. 4.

Fibrous elements, i.e., fibrils, of protein may be provided through mechanical disintegration of the protein raw material, such as material comprising collagen fibers. The protein fibers are mechanically divided so as to form protein or collagen fibrils depending on the application. Mechanical disintegration means any means for disintegration or fibrillation of protein fibers to obtain protein fibrils, such as collagen fibrils. Fibrillation may generally within the scope of the invention be carried out, for example, using a stone mill, refiner, grinder, homogenizer, colloider, supermass colloider, friction grinder, ultrasound-sonicator, fluidizer, such as microfluidizer, macrofluidizer or fluidizer-type homogenizer. In an example, collagen fiber suspension may be refined using Masuko grinder.

Prior to the mechanical disintegration, also referred to as mechanical dividing, the natural fibers being the source of the fibrils may be chemically treated, e.g., by using swallowing agents reducing the energy consumption of the subsequent process.

The suspension may comprise water, fibrils of natural protein, and at least one additive.

The fibrous suspension is directed through a nozzle so as to form a fibrous filament. The nozzle may feed the fibrous suspension to a surface. The surface may be a surface of a belt or of a cylinder. The fibrous suspension is dried on the surface. Drying removes water from the fibrous suspension. The dried fibrous suspension is arranged to form a fibrous filament onto the surface. The fibrous filament may be continuous. The continuous fibrous filament may be extracted from the surface. The fibrous filament extracted from the surface may be cut or shortened in order to form staple fibers.

Alternatively, the fibrous suspension fed to the surface is arranged to be shortened and dried on the surface. The dried and shortened fibrous suspension is arranged to form staple fibers. This is enabled by surface structure, such as grooves arranged on a curved surface. The shortened fibers are extracted from the surface.

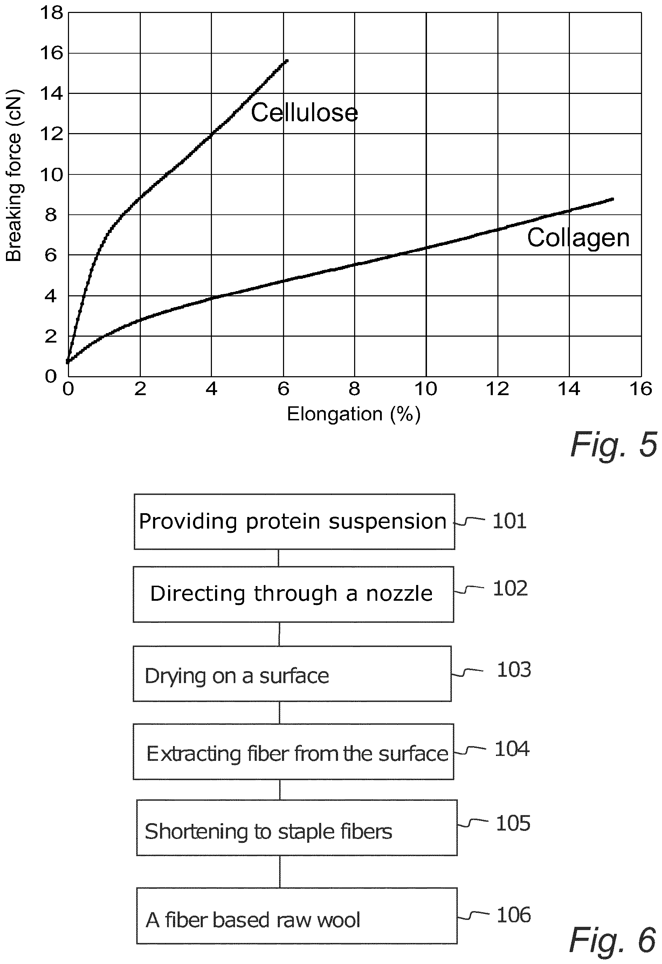

Fig. 6 illustrates a method for manufacturing a natural fiber based raw wool according to an embodiment. A protein suspension is provided 101. The protein suspension comprises aqueous suspension of mechanically divided protein fibrils. In other words, the protein fibrils as such are to be regarded as natural fibril. The protein suspension may comprise water, divided protein fibrils and at least one rheology modifier. Fibrils of the protein suspension may originate from mechanically shortened protein fibers. In a preferred embodiment of the invention, the protein fibrils are collagen fibrils originating from tanned hide-based material. The collagen fibrils may thus originate from e.g., wet-blue, i.e., hides which has been tanned and where the collagen fibers used as a basis for the reconstructing of a collagen-based filament has been pretreated e.g. by chrome according to conventional and well-known tanning methods.

The protein suspension is directed through a nozzle 102. The nozzle feeds the protein suspension to a surface. The surface may be a surface of a belt or of a cylinder. The protein suspension is dried on the surface 103. Drying removes water from the protein suspension. The dried protein suspension is arranged to form a reconstructed fiber on the surface. The reconstructed fiber may be arranged in a form of a continuous reconstructed fiber. The continuous reconstructed fiber is extracted from the surface 104. The reconstructed fiber extracted from the surface is cut or shortened in order to form staple fibers 105. The stable fibers are arranged to form an inhomogeneous network comprising fiber concentrations of varying density and orientation. The inhomogeneous fluffy material of staple fibers is called a natural fiber based raw wool 106.

The above process is described as a process using collagen fibers as a source for the manufacture of staple fibers.

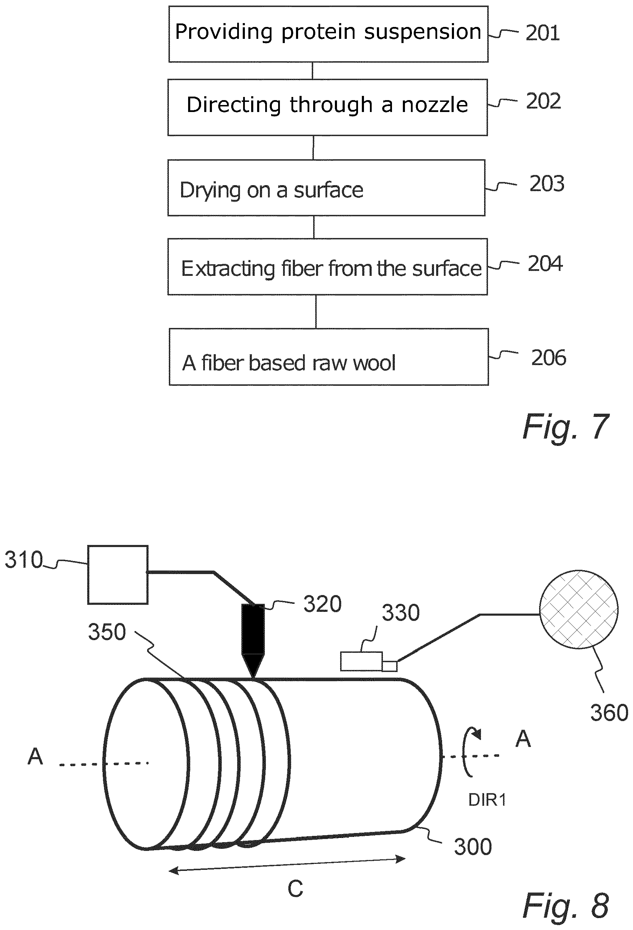

Fig. 7 illustrates a method for manufacturing a natural fiber based raw wool according to an embodiment. A protein suspension is provided 201. The protein suspension comprises aqueous suspension of divided protein fibrils. The protein suspension may comprise water, at least one rheology modifier and divided protein fibrils. Divided protein fibrils may originate from tanned hides, which has been mechanically subdivided into pulp. The protein suspension is directed through a nozzle 202. The nozzle feeds the protein suspension to a surface, for example on a surface of a belt or of a cylinder. The protein suspension is arranged to be shortened and dried on the surface 203. The dried and shortened protein suspension is arranged to form staple fibers. This is enabled by grooves (not shown) arranged on a surface. In following embodiments, grooves refer to structures enabling a formation of staple fibers having a length defined by the distance between the grooves. Those grooves, including grooves 401 and 601 may generally alternatively be made as ridges performing the same function, i.e., weakening strength of the reconstructed filament at positions where the filament may divide or break into the desired reconstructed staple fibers. The shortened fibers are extracted from the surface 204. The stable fibers are arranged to form an inhomogeneous network comprising fiber concentrations of varying density and orientation. The inhomogeneous fluffy material of staple fibers is called a natural fiber based raw wool 206.

A protein suspension could comprise collagen fibrils. Collagen fibrils are natural fibrils originating from shavings, leather waste, pulp and/or tanned hides including the hazardous waste material from the tanning process. The collagen fibrils are thus originating from different animals, typically skin. The shavings may originate from wet blue, wet white, leather shavings or tanned hides.

Collagen may have macromolecular structure repeating amino acid sequences (GPX)n and glycine(G)-X-hydroxyproline(HYP). (GPX)n is the most common amino acid sequence in collagen. In the sequence X refers to any amino acid other than glycine, proline or hydroxyproline. The amino acid composition of collagen is atypical for proteins, particularly with respect to its high hydroxyproline content. Collagens are the most abundant proteins found in tissues of animals. Collagen is main structural protein in extracellular space in the connective tissues including tendons, ligaments and skin. There are several types of collagen. Type I (ski, tendon, bone), II (hyaline cartilage), III (reticulate), V (cell surfaces, hair, placenta) and XI (cartilage) being fibrillar. A single collagen molecule, tropocollagen, is used to make up larger collagen aggregates, such as fibrils. A collagen molecule is approximately 300 nm long and 1.5 nm in diameter. It is made up of three polypeptide strands (called alpha peptides), each of which has the conformation of a left-handed helix. These three left-handed helices are twisted together into a right- handed triple helix or "super helix", a cooperative quaternary structure stabilized by many hydrogen bonds. With type I collagen and possibly all fibrillar collagens, if not all collagens, each triple-helix associate into a right-handed super-super-coil referred to as the collagen microfibril. Each microfibril is interdigitated with its neighboring microfibrils.

If collagen is irreversibly hydrolyzed (e.g., by heat and chemicals), it is termed gelatin. Gelatin is modified protein.

Collagen fibrils may be in native form, which have not undergone any chemical modification. Natural collagen fibers and natural collagen fibrils may be non-regenerated. Thus, natural collagen fibers/fibrils have not undergone chemical regeneration or physical modification of the collagen polymer structure. It should nevertheless be noted that the collagen fibrils may have been chemically or at least physically affected when the source of collagen fibrils are tanned hides of animals.

Mechanical disintegration into protein fibrils from protein raw material, protein pulp, or refined pulp is carried out with suitable equipment such as a refiner, grinder, homogenizer, colloider, friction grinder, ultrasound sonicator, fluidizer such as microfluidizer, macrofluidizer or fluidizer-type homogenizer.

In particular and advantageously mechanical disintegration into collagen fibrils from collagen raw material, collagen pulp, or refined collagen pulp is carried out with suitable equipment such as a refiner, grinder, homogenizer, colloider, friction grinder, ultrasound sonicator, fluidizer such as microfluidizer, macrofluidizer or fluidizer-type homogenizer.

Protein fibers, such as collagen fibers may be isolated from any relevant protein containing raw material using chemical-, mechanical-, bio-, thermo-mechanical-, or chemi-thermo-mechanical pulping process. Mechanically shortened, divided or cut fibers may comprise chemically or physically modified derivative of collagen micro fibrils or fibril bundles.

A protein suspension may comprise 80-98 wt-% of water and 2-20 wt-% of protein. The protein suspension may comprise 85-98 wt-% of water and 2-15 wt% of protein. In addition, the protein suspension may comprise 0-5 wt-% of rheology modifier.

A collagen suspension may comprise 80-98 wt-% of water and 2-20 wt-% of collagen. The collagen suspension may comprise 85-98 wt-% of water and 2-15 wt% of collagen. In addition, the collagen suspension may comprise 0-5 wt-% of rheology modifier.

The mechanically divided protein fibril may be pure protein structures or comprise chemically modified or chemically treated protein fibrils. Thus, the protein suspension comprises mechanically divided or shortened protein fibers. According to a preferred embodiment of the invention, the applied protein source is collagen.

Fiber product may include additives like rheology modifier(s), binder(s), cation active reagent(s), crosslinking agent(s), dispersion agent(s), pigment(s), and/or other modifier(s).

Fiber and staple fibers may comprise additives such as alginate, alginic acid, pectin, carrageenan, anionic polyacrylamide (APAM), cationic polyacrylamide (CPAM), polyethylene oxide (PEO), carboxymethyl cellulose (CMC), starch, enzymes (such as laccase, transglutaminase), polyphenols, glutaraldehyde, gelatine, casein, glucose, Tris-HCl, phosphate or a combination of such.

Rheology modifier comprises a compound or agent arranged to modify the viscosity, yield stress and/or thixotropy of the suspension. Rheology modifier may comprise high molecular weight polymers. Rheology modifier is arranged to modify protein suspension rheology by adjusting gel strength and yield point of the protein suspension.

Fig. 8 illustrates an apparatus for manufacturing natural fiber based raw wool according to an embodiment. A protein suspension 310 is fed to a nozzle 320. The refined fibrils of the protein suspension align in a nozzle. The aqueous suspension of water and fibrils of the protein suspension form an oriented fibril network in a nozzle 320.

The protein suspension 310 is directed onto a cylinder 300 via the nozzle 320. The protein suspension exiting the nozzle 320 is shaped according to the nozzle outlet opening. The nozzle outlet opening may be shaped as a round or as elliptic, for example. The protein suspension exits the nozzle 320 in a form of a continuous strand having a cross-sectional shape corresponding to that of the nozzle outlet opening. The protein suspension exiting the nozzle 320 may have round or elliptic cross-sectional shape.

The nozzle 320 may be arranged to move back and forth along the longitudinal direction C, i.e., direction of the rotational axis A, of the cylinder 300, along horizontal plane. The protein suspension 310 is directed to a certain horizontal level along the longitudinal cylinder surface. Due to rotation of the cylinder 300 and movement of the nozzle 320, the fed protein suspension 310 circles around the cylinder surface forming a round next to a round, or partly overlapping with adjacent rounds. The continuously injected protein suspension 310 moves on the surface of the cylinder 300 with the rotating cylinder 300. While the cylinder 300 rotates around its rotational axis A, the nozzle 320 is arranged to move to an adjacent place along longitudinal cylinder surface C.

Alternatively, two or more nozzles may be arranged adjacent, parallel along longitudinal cylinder surface C. The two or more nozzles are united, forming an integrated unit, and arranged to move concurrent along longitudinal cylinder surface C. The distance between two adjacent nozzles may be in order of centimeters, for example 1 cm. The nozzle(s) are oscillating along the longitudinal dimension of the cylinder. In case of two or more nozzles arranged at 1 cm distance from each other, the time that the nozzles take to move 1 cm corresponds to drying time of the protein suspension injected on the cylinder surface. Moving speed of nozzles is arranged such that time for a length of the dimension between the two adjacent nozzles is arranged to correspond to drying time of the protein suspension.

During drying water is removed and fibrils star forming hydrogen bonds. Thereby fiber is formed. Hydrogen bonds are formed as dry content increases from 70 wt-% towards 100 wt-%. Dried protein suspension or fiber forming or fiber refer to dry content of at least 70 wt-%. Fiber yarn count comprises 1-20 dtex. Fiber diameter may be 15-70 µm. Fiber tensile strength may be 10-25 cN/tex, preferably 15-20 cN/tex, and comprise stretch or elongation break of 10-30 %, preferably 15-25%.

In case of multiple nozzles along the longitudinal dimension of the cylinder, the multiple nozzles may be placed next to each other along the whole longitudinal dimension of the cylinder. Thus, there is no need for movement of nozzles, but those may be fixed in their places. In this case the drying time of the protein suspension corresponds to time of rotation of the cylinder.

With two or more nozzles, the cylinder is covered with protein suspension and fiber is formed faster than in case of one nozzle. Accordingly extracting fibers, providing oil and other relating functions shall be accomplished at corresponding pace.

The protein suspension exits from a nozzle and/or injection onto a surface may be controlled hydraulically or pneumatically. Velocity of the protein suspension exiting the nozzle may be controlled by pressure applied on the protein suspension at the nozzle.

An oil or wax supply 330 may be arranged relative to a surface of the cylinder 300. The oil supply 330 is arranged to move, in an oscillating manner, as the nozzle 320, along longitudinal dimension of the cylinder 300, parallel with the axis of rotation A of the cylinder 300, at certain vertical level. In case of oscillating nozzle(s) 320 the oil supply 330 is arranged to oscillate simultaneously with the nozzle(s) 320.

Rotation of a cylinder may be controlled externally, for example by an electric motor, whose rotational speed is adjustable. A cylinder or a curved belt may generate a centripetal acceleration of 1-1000 g, preferably 100-500 g. Diameter of a cylinder may be 1-6 m. Rotational speed of the surface of the cylinder may be 5-25 m/s. The centripetal force (Fcp) acting on suspension (m) is dependent on radius (r) of the cylinder and its rotational surface speed (v). The centripetal force (Fcp) acting on suspension (m) is dependent on radius (r) of curvature of a belt and its rotational surface speed (v). Mathematically: Fcp=ma=mv2/r; wherein a=v2/r.

The protein suspension is dried on the surface of the cylinder 300. This may be effected internally and/or externally. Heating internally may be effected via electric heating resistor, heating steam or air. Heating externally may be effected via irradiation, heating and/or air blow. The dried protein suspension forms a fiber 350 onto surface of the cylinder 300. The fiber 350 is extracted from the surface of the cylinder 300. Extracting may be based on blowing, suction, vacumization, scraping or dropping fibers from the surface based on gravitation. The fiber 350 may be extracted mechanically or using vacuum or pressurized air. The extraction may be implemented manually or automatically. An extractor may be placed on a side of the cylinder 300 opposite to the nozzle 320. The extractor may have a fixed place, or the extractor may be arranged to move, in an oscillating manner, as the nozzle 320, along longitudinal dimension of the cylinder 300, parallel with the axis of rotation A of the cylinder 300, at certain vertical level. The extractor is arranged to oscillate simultaneously with the nozzle. The extractor may be integrated with the oil supply 330 and move with it. Oil is supplied on a cylinder surface after the fiber has been extracted from the cylinder surface.

The extracted fiber 360 is in form of unoriented and entangled fiber based raw wool, which may comprise uneven clumps among fluffy fiber based raw wool. When the fiber based raw wool comprises continuous fiber, the length of fiber is arranged to be cut or shortened to form staple fibers. After shortening fiber based raw wool comprising staple fibers is formed.

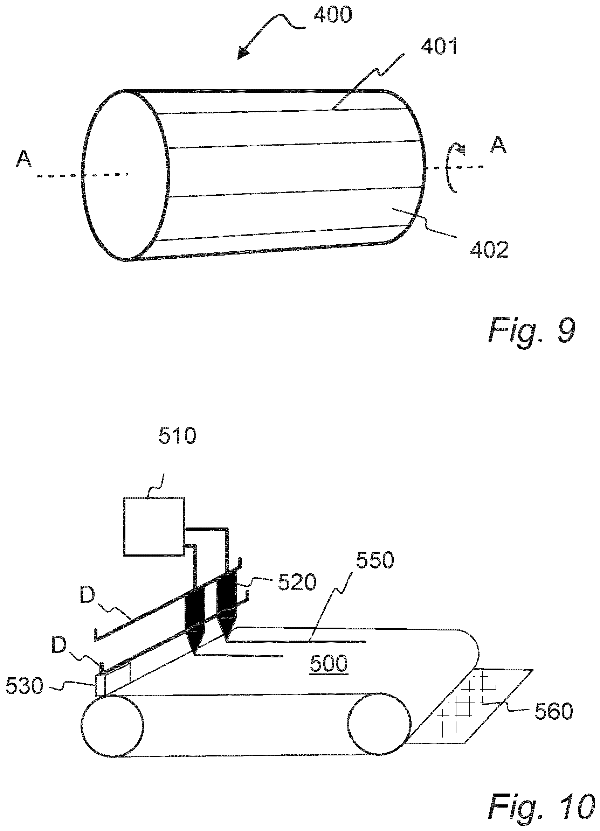

Fig. 9 illustrates a cylinder according to an embodiment of the invention. Axis of rotation is A of the cylinder 400. Cylinder may comprise an even, flat external surface or a grooved surface, as illustrated. Grooves 401 are arranged onto external surface of the cylinder 400 parallel with the axis of rotation A of the cylinder 400. The injected protein suspension is in contact with ridges 402 between the grooves 401 of the cylinder surface. Grooves 401 form a weak point for the protein suspension and have effect of forming a discontinuity for the continuously injected protein suspension. The continuously injected protein suspension has a break at each groove. Thereby the protein suspension forms separate parts of length of ridges 402 between the grooves 401. After extensive water is dried, staple fiber is formed onto the cylinder, on the ridges 402 between the grooves 401. Length of formed staple fibers is determined by the length between grooves 401 on the cylinder surface.

In case of grooved cylinder, the fiber based raw wool extracted from the cylinder comprises staple fibers. No additional refining, shorting or cutting means or phases are needed. The staple fiber based raw wool is processable. The fiber based raw wool comprises staple fibers, which have been shortened to a predefined length with the aid of grooves. The spacing between the grooves on the cylinder surface determine length of the staple fibers.

Fig. 10 illustrates an apparatus for manufacturing natural fiber based raw wool according to an embodiment. A protein suspension 510 is fed to a one or more nozzle 520. The refined fibrils of the protein suspension align in a nozzle. The fibrils of the protein suspension form an oriented fibril network in a nozzle 520.

The protein suspension 510 is directed onto a belt 500 via the nozzle(s) 520. The protein suspension exiting the nozzle 520 is shaped according to the nozzle outlet opening. The nozzle outlet opening may be shaped as a round or as elliptic, for example. The protein suspension exits the nozzle 520 in a form of a continuous strand having a cross-sectional shape corresponding to that of the nozzle outlet opening. The protein suspension exiting the nozzle 520 may have round or elliptic cross-sectional shape.

Two or more nozzles may be arranged adjacent, parallel along cross- sectional or transverse belt surface. Cross/transverse dimension refers to a width dimension of the belt; perpendicular to a longitudinal dimension of the belt, which corresponds to the moving direction of the belt. The two or more nozzles may be united, form an integrated unit. The distance between two adjacent nozzles may be in order of 0.5-50 mm, or 0.5-20 mm, for example 1 mm. The multiple nozzles may be placed next to each other along the whole cross dimension of the belt. In such case the whole cross dimension of the belt is covered with injected protein suspension at the same time via multiple nozzles.

During drying water is removed and fibrils star forming hydrogen bonds. Thereby fiber is formed. Hydrogen bonds are formed when dry content is from 70 wt-% to 100 wt- %. Dried protein suspension or fiber forming or fiber refer to dry content of at least 70 wt-%.

The protein suspension exits from a nozzle and/or injection onto a surface may be controlled hydraulically or pneumatically. Velocity of the protein suspension exiting the nozzle may be controlled by pressure applied on the protein suspension at the nozzle.

An oil or wax supply 530 may be placed before the nozzle(s) 520 in relation to the moving direction of the belt 500. The oil supply 530 may be arranged to move, in an oscillating manner along cross dimension of the belt 500, perpendicular with direction of movement of the belt 500. The oil supply 530 is arranged after fiber extraction phase. Oil is supplied on the surface of the belt 500. The protein suspension is injected on an oily belt surface.

The protein suspension is dried on the surface of the belt 500. This may be effected internally and/or externally. Heating internally may be effected via the belt, e.g., by an electric heating resistor, heating steam or air. Heating externally may be effected via irradiation, heating and/or air blow. The dried protein suspension forms a fiber 550 onto surface of the belt 500. The fiber 550 is extracted from the surface of the belt 500. Extracting may be based on blowing, suction, vacumization, scraping or dropping fibers from the surface based on gravitation. The fiber 550 may be extracted mechanically or using vacuum or pressurized air. The extraction may be implemented manually or automatically. An extractor may be placed on any part of the belt 500, where the dryness of the fiber is at a desired level, for example over 70 wt-%. The extractor may be placed at end of the belt, or at either external side of the belt, wherein upper external side of the belt is arranged to move at opposite direction than the lower external side of the belt. It is possible to convey protein suspension or fiber on another belt for further drying.

Oil is supplied on a belt surface before the protein suspension is injected onto the surface.

The extracted fiber 560 is in form of unoriented and entangled fiber based raw wool, which may comprise uneven clumps among fluffy fiber based raw wool. When the fiber based raw wool comprises continuous fiber, the length of fiber is arranged to be cut, shortened, or refined to form staple fibers. After shortening fiber based raw wool comprising staple fibers is formed.

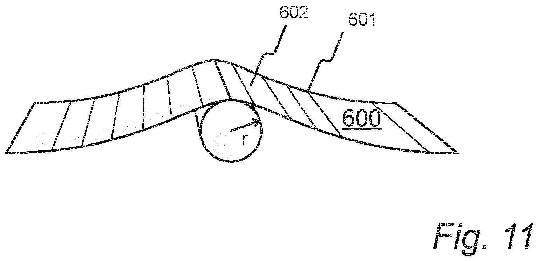

Fig. 11 illustrates a belt according to an embodiment of the invention. It shows a belt 600 comprising curved and grooved surface. The belt 600 may be used for forming staple fibers, while in case of even, flat, non-curved belt a continuous fiber is formed on the belt. The curved surface of the belt comprises a radius of curvature of 0.25-4 m.

Grooves 601 or ridges are arranged onto external surface of the belt 600. The grooves 601 are arranged along a transverse direction of the belt 600, perpendicular to the longitudinal dimension or direction of movement of the belt 600. The injected protein suspension is in contact with ridges 602 between the grooves 601 of the belt surface. Grooves 601 or ridges form a weak point for the protein suspension and have effect of forming a discontinuity for the continuously injected protein suspension. The continuously injected protein suspension has a break at each groove. Thereby the protein suspension forms separate parts of length of ridges 602 between the grooves 601. After extensive water is dried, staple fibers are formed onto the belt, on the ridges 602 between the grooves 601. Length of formed staple fibers is determined by the length between grooves 601 on the belt surface.

In case of grooved belt, the fiber based raw wool extracted from the belt comprises staple fibers. No additional refining, shorting or cutting means or phases are needed. The staple fiber based raw wool is processable. The fiber based raw wool comprises staple fibers, which have been shortened to a predefined length with the aid of grooves. The spacing between the grooves on the belt surface determine length of the staple fibers.

When the continuous fiber is shortened as staple fibers, cut end of a staple fiber is sharp, or at least substantially sharp. When staple fiber is formed on a curved, grooved surface by the grooves, the end of the staple fiber is uneven or irregular in comparison to the sharp cut end of a staple fiber. The groove causes interruption to the applied continuous protein suspension, thereby forming separated portions of certain length, which, after dried, form staple fibers. Such interrupted surface of a staple fiber may show fibrils or smaller portions. In an extended view, uneven end surface of interruption shows irregular shape. In principle, a ridge would be able to perform the same function, namely dividing the continuous fiber, i.e., the filament, into separate staple fibers.

The surface, of e.g., a belt or a cylinder, may comprise hydrophobic surface material. External surface of a cylinder or a belt may be covered with a nonporous hydrophobic polymer coating. The polymer coating has effect of forming a hydrophobic surface. The hydrophobic polymer coating may be covered with oil. The coating and oil coverage of the surface enable to achieve a hydrophobic, low friction and low contact angle hysteresis external surface. The oil effects on protein suspension remaining in contact with the external surface via the oil. The polymer coating and oil on the surface and/or centripetal force of a rotating cylinder enable controlling and maintaining round cross-sectional shape of the protein suspension injected on the surface. Oil and low contact angle hysteresis on the outer surface of the cylinder or the belt have effect on maintaining the round cross-sectional shape of the protein suspension during drying. Oil has effect of reducing risk of the protein-solution adhering to the surface. Oil has effect of providing stable cross-sectional shape fiber and/or avoid formation of unwanted wide, thin and/or weak ribbons on the surface. Part of the oil remains with the fiber and acts as finishing agent. Oil may be used for example to control friction between fibers and/or between fibers and metal during processing. These are desired parameters for processing the fiber based raw wool during further processing and formation of products, like yarn, non-woven or other.

The protein suspension directed through a nozzle onto a surface is dried on the surface. Drying is accomplished via heat and, in case of a cylinder, a rotating motion of a cylinder. Rotation of the cylinder and/or heating enable drying of provided protein suspension on the surface. A continuous fiber may be formed on the surface. Alternatively, stable fibers may be formed on the surface. The protein suspension may be dried externally and/or internally via the surface of a cylinder or of a belt. The cylinder or the belt may comprise a heating element. The cylinder or the belt may comprise an internal heater. The internal heater may implement heating electronically, via a resistor, or via hot steam. The cylinder or the belt may comprise an external heater. The external heater may provide irradiation or air blow or steam blow towards the surface in order to dry the protein suspension on the surface. The heater(s) enable drying protein suspension applied on the surface. Heater or heating elements are arranged to dry the protein suspension by removing water.

Oil is applied on the surface. Oil on the surface has effect of reducing surface tension, friction and/or surface contact angle hysteresis. Oil may comprise, but is not limited to, fiber finishing oils to reduce fiber-fiber friction or fiber-metal friction, or a vegetable oil or a non-immiscible fluid.

The surface is arranged to move and convey the injected protein suspension. The surface may comprise grooves. Staple fibers may be formed on a curved and grooved surface. The surface may comprise a curved or round cylinder surface, or a curved belt surface. The grooves are aligned transverse to the direction of movement of a surface. Grooves are arranged transverse to the direction of movement of a belt. The grooves extend along transverse direction of the belt, which is perpendicular to the longitudinal dimension, or direction of movement of the belt. Grooves are arranged transverse to the direction of rotation of a cylinder. The grooves extend along length direction of the cylinder, being in parallel with the axis of rotation of the cylinder. The surface comprises thin grooves, in comparison to wide ridges between the grooves. The ridges of the surface form support surface for injected protein suspension, while grooves form a discontinuation places. Grooves and the ridges between them enable forming staple fibers on the surface. Injected protein suspension is arranged to break at a groove. Spacing between the grooves is configured to define length of formed staple fibers.

Width of the grooves may be 0.5-5 mm, preferably 2-3 mm. Depth of the grooves may be 0.5-10 mm, preferably 2-3 mm.

Centripetal force of a moving surface, hydrophobic properties of a surface and oil properties have effect on preserving round cross section of the injected protein suspension and fiber, as its dried form, on the surface.

As free water is removed from the protein suspension during drying, hydrogen bonds start to appear. This occurs on/after solid content of the protein suspension exceeds fiber content of 70 wt-%.

The dried protein solution forms a continuous fiber (strand) onto a continuous surface. Grooved and curved surface enable forming of stable fibers without additional refining or shortening after drying. The staple fibers comprise length of 6-80 mm, preferably 30-70 mm.

Long fibers or staple fibers may be interlocked together in order to form a permanent network of fibers. Disintegration of the hydrogen bonds may be done by exposing the staple fibers to water or aqueous solution. A minor mechanical or hydrodynamical force, like a pull or a twist or a hydrodynamic shear, disintegrates a wetted staple fiber composition or product. When exposed to water, the staple fibers will return into separate primary protein fibrils. This enables forming water disposable products.

The natural fiber based raw wool comprises large specific surface area and low density. It provides good filtering properties and a good insulator, while its thermal conductivity is low. Due to uneven fiber surface, the natural fiber based raw wool comprises high friction, which is desired property for further processing, for example manufacturing non-woven material.

The natural fiber based raw wool comprises good water absorption and water retention properties. Water retention of the natural fiber based raw wool may be 10-100 times its own weight.

The fiber based raw wool may be processed as a raw wool sheared from a sheep. The fiber based raw wool comprises staple fibers. The fiber based raw wool comprises staple fibers in fluffy arrangement, in unorganized, unoriented order and forming clumps or conglomerates of different densities.

The fiber based raw wool may be carded. Generally carding orients, detangles and cleans raw wool towards oriented strands. Carding may be performed by a card, by a carding machine, by heckling machine. Carding machine may have surface covered with carding clothing or soft-bristled brush attachment. During carding the staple fibers orientate towards common fiber orientation and the staple fiber density becomes more even, while reducing clumps. Due to carding fibers tend to orient similarly, thereby having substantially similar orientation among longitudinal dimension of fibers. The carded fiber based raw wool comprises at least mainly oriented staple fibers and density variations or clumps are reduced. The structure tends to become more homogenous during carding.

Natural fiber based raw wool has properties desired during carding, like low friction between fibers and/or between fibers and metal (of cards).

The carded fiber based raw wool may be processed as a yarn or as a non- woven material. Yarn may be made from carded fiber based raw wool by forming a continuous pre-yarn and spinning several pre-yarns as a yarn. The yarn may be used for manufacturing textiles of different kind. A textile may be made of yarn using known textile manufacturing processes and equipment.

The non-woven material may be produced from the carded fiber based raw wool by a non-woven process, or felting, for example needle punch, hydro-entanglement or other suitable method. The non-woven process binds the staple fibers mechanically in order to produce continuous non-woven material or fabric. Adhesive may be added in order to enhance bonding.

Non-woven material may be used to manufacture hygiene products. The hygiene products may be disposed with water, whereby the product breaks down to protein fibrils. The hygiene products may comprise flushable products, which disintegrate into water. Hygiene products may comprise wipes or diapers. The products made of the staple fiber based raw wool have firm fabric and feel, when those are dry. However, underlying fibrils are small and locked together via hydrogen bonds, which become very weak at aqueous environment. Once in water, even a low shear will cause staple fibers to disintegrate back to sorter protein fibrils. In addition to disposability, the non-woven material made of staple fiber based raw wool has ability to absorb and retain water. This ability is desired for products like diapers and alike.

Yarn made of natural fiber based raw wool may have yarn count of 5-200 tex. The yarn comprises tenacity of 5-15 cN/tex; and elongation at break of 3-10 %. Non-woven material made of natural fiber based raw wool comprises density of 10-100 kg/m3.

The manufacturing process enables providing yarn and/or non-woven material economically and environmentally friendly way. The provided fiber based raw wool is provided by compact manufacturing phase, even as a single process. The fiber based raw wool is processable with process and equipment known from raw wool processing and handling. The fiber based raw wool may be processed as yarn using yarn spinning equipment or as non-woven material using non-woven process and method.

The staple fiber based raw wool or products have effect on biodegradability. Discarding is ecological and use of natural based cellulosic fibers enables recycling and reuse.

With reference to fig. 2, an example of the mechanical properties of the fiber filament manufactured according to one of the embodiments of fig. 6-11 are shown. The two compared fibers are reconstructed on the basis of natural fiber sources and additive(s). The fiber referred to as "cellulose" comprises 90% of fibrils of natural cellulose and 10% of CMC as an additive. The fiber product referred to as "protein" comprises 90% of fibrils of natural collagen and 10% of CMC as an additive.

Stretch of the protein-based fiber being above 10% and cellulose fiber product being less than 10% shows the protein-based fiber having higher elasticity. In turn, maximum tenacity of the cellulose-based fiber is above 20 cN/TEX and the protein based maximum tenacity is less than 15 cN/TEX, which shows the cellulose-based fiber tested has higher tensile strength.

With reference to fig. 5, an example of the mechanical properties of reconstructed fiber products according to e.g., one of the embodiments of fig. 6-11, are shown. Fiber product referred to as "cellulose" comprises 85% of fibril of natural cellulose and 15 % of additive(s). Fiber product referred to as "collagen" comprises 85% of fibrils of collagen and 15 % of additive(s). Linear mass density of the fiber products is 7.5 dtex. Elongation of the fiber product of cellulose is about 6%. Elongation of the fiber product of collagen is about 15%. Breaking force of the fiber product of 30 cellulose is above 14 cN. Breaking force of the fiber product of collagen is above 8 cN.

Fig. 3 illustrates a plurality of staple fibers produced according to one of the embodiments of fig. 6-11.

A reconstructed fiber product may also be produced according to e.g., US patent application 2017/356102, WO application 2016/174306, or WO 2016/174307, hereby incorporated by reference.

According to an example, a yarn comprising staple fibers including 85% of fibrils of natural collagen and having linear mass density of 7.5 dtex has elongation of at least 10 %, for example 15%.

## Claims

1. A reconstructed filament wherein the reconstructed filament comprises natural protein fibrils and an additive.

2. A reconstructed filament according to claim 1, wherein the reconstructed filament comprises natural protein fibrils and an additive, wherein the reconstructed filament comprises natural protein fibrils in an amount of between 70 and 99.9 % by weight of the filament and an additive between 0.1 and 30 % by weight of the filament.

3. A reconstructed filament according to claim 1 or 2, wherein said protein fibrils comprise elastin fibrils.

4. A reconstructed filament according to any of the previous claims, wherein said protein fibrils comprise resilin fibrils.

5. A reconstructed filament according to any of the previous claims, wherein said protein fibrils comprise keratin fibrils

6. A reconstructed filament according to any of the previous claims, wherein said protein fibrils comprise collagen fibrils obtained from natural collagen fibers.

7. A reconstructed filament according to any of the previous claims, wherein the natural protein fibrils originate from mechanically subdivided natural protein fibers.

8. A reconstructed filament according to any of the previous claims, wherein the natural protein fibrils originate from mechanically subdivided natural protein fibers.

9. A reconstructed filament according to any of the previous claims, wherein the natural protein fibrils comprise fibrils of non-tanned hides.

10. A reconstructed staple fiber manufactured on the basis of a reconstructed filament according to any of the claims 1-9.

11. A reconstructed staple fiber according to claim 10, wherein the lengths of the originating natural protein fibers are different from the length of the staple fiber.

12. A wool comprising a plurality of staple fibers according to any of the claims 10 or 11, wherein the natural protein fibrils originate from mechanically subdivided natural protein fibers.

13. A yarn spun from staple fibers of reconstructed filament according to any of the claims 1-9 and/or a yarn spun from staple fibers according to claims 10 or 11.

14. A yarn according to claim 13 comprising a plurality of spun staple fibers based on natural collagen, wherein the yarn comprises collagen staple fibers having a length of at least 10 mm.

15. A yarn according to any of the claims 13 or 14, wherein the yarn comprises collagen staple fibers having a length of 6-80 mm.

16. Use of the filament, the staple fiber and/or the yarn according to any of the claims 1-15 in textile and/or shoe industry.

17. An item comprising the filament, the staple fiber and/or the yarn according to any of the claims 1-15, the item comprising fabric, woven material, nonwoven material, clothing or footwear product.
